Europäisches Patentamt

European Patent Office

Office européen des brevets

(11) Veröffentlichungsnummer: **0 519 436 A1**

# EUROPÄISCHE PATENTANMELDUNG

(21) Anmeldenummer: **92110247.1**

(22) Anmeldetag: **17.06.92**

(51) Int. Cl.5: **C07C 67/055**, C07C 69/15, B01J 23/58

(30) Priorität: **21.06.91 DE 4120491**

(43) Veröffentlichungstag der Anmeldung:
**23.12.92 Patentblatt 92/52**

(84) Benannte Vertragsstaaten:
**DE ES FR GB IT NL**

(71) Anmelder: **HOECHST AKTIENGESELLSCHAFT**
**Postfach 80 03 20**
**W-6230 Frankfurt am Main 80(DE)**

(72) Erfinder: **Wirtz, Peter, Dr.**
**Wiesbadener Strasse 135**
**W-6240 Königstein/Ts.(DE)**
Erfinder: **Wörner, Karl-Fred, Dr.**
**Georg-Büchner Strasse 27**
**W-6236 Eschborn(DE)**
Erfinder: **Wunder, Friedrich, Dr.**
**Rosenpark 22**
**W-6234 Hattersheim/M.(DE)**
Erfinder: **Eichler, Klaus, Dr.**
**Im Traminer 10**
**W-6236 Eschborn(DE)**
Erfinder: **Roscher, Günter, Dr.**
**Hölderlinstrasse 64**
**W-6233 Kelkheim (Taunus)(DE)**

(54) **Katalysator und Verfahren zur Herstellung von Vinylacetat.**

(57) Die Erfindung betrifft ein Verfahren zur Herstellung von Vinylacetat in der Gasphase aus Ethylen, Essigsäure und Sauerstoff oder Sauerstoff enthaltenden Gasen an einem Katalysator, der Palladium und/oder dessen Verbindungen sowie Alkaliverbindungen auf einem Träger enthält, wobei der Katalysator außerdem mindestens eine Bariumverbindung enthält und Gold, Cadmium und deren Verbindungen abwesend sind. Weiter betrifft die Erfindung den genannten Katalysator.

EP 0 519 436 A1

Es ist bekannt, daß man Ethylen in der Gasphase mit Essigsäure und Sauerstoff oder sauerstoffhaltigen Gasen an Festbettkatalysatoren zu Vinylacetat umsetzen kann. Katalysatoren, für die eine Raum-Zeit-Leistung von mehr als 200 g/l•h angegeben wird, enthalten ausnahmslos eine der beiden Element-Kombinationen Palladium/Cadmium/Kalium oder Palladium/Gold/Kalium (US-PS 3 939 199, US-PS 4 668 819, EP-A-0 330 853, EP-A-0 403 950, EP-A-0 431 478).

Überraschenderweise wurde nun gefunden, daß Palladium-Katalysatoren, die mindestens eine Bariumverbindung, und mindestens eine Alkalimetallverbindung, aber weder Cadmium noch Gold oder deren Verbindungen enthalten, eine mindestens gleiche Raum-Zeit-Leistung, eine gleiche oder niedrigere Ver-brennung von Ethylen zu $CO_2$ und eine gleiche oder niedrigere Ethylacetat-Bildung bewirken als die genannten Katalysator-Systeme Pd/Au/K und Pd/Cd/K. Dies ist sehr vorteilhaft, denn Cadmium ist wegen seiner Giftigkeit und Gold wegen seines hohen Preises nachteilig, wogegen Barium preiswert und in der Natur ungiftig ist, da es sofort in Bariumsulfat übergeht, das wegen seiner Schwerlöslichkeit völlig inert ist.

Gegenstand der Erfindung ist demgemäß ein Verfahren zur Herstellung von Vinylacetat in der Gasphase aus Ethylen, Essigsäure und Sauerstoff oder Sauerstoff enthaltenden Gasen an einem Katalysator, der Palladium und/ oder dessen Verbindungen sowie Alkaliverbindungen auf einem Träger enthält, dadurch gekennzeichnet, daß der Katalysator mindestens eine Bariumverbindung enthält und daß Gold und Cadmium und deren Verbindungen abwesend sind.

Ein weiterer Gegenstand der Erfindung ist ein Katalysator, der Palladium und/oder dessen Verbindungen sowie Alkaliverbindungen auf einem Träger enthält, dadurch gekennzeichnet, daß der Katalysator mindestens eine Bariumverbindung enthält und daß Gold und Cadmium und deren Verbindungen abwesend sind.

Abwesenheit dieser Elemente und ihrer Verbindungen bedeutet dabei, daß sie höchstens in Spurenmengen, aufgrund von Verunreinigungen der Ausgangsstoffe des Trägerkatalysators, anwesend sind, d.h. in Gesamtkonzentrationen von Cadmium und Gold von höchstens 1 ppm, bezogen auf den gesamten Trägerkatalysator (Träger plus aktive Komponenten).

Als Träger eignen sich die bekannten inerten Trägermaterialien wie Kieselsäure, Aluminiumoxid, Alumosilikate, Silikate, Titanoxid, Zirkonoxid, Titanate, Siliciumcarbid und Kohle. Besonders geeignet sind Kieselsäuren mit einer spezifischen Oberfläche von 40 bis 350 $m^2$/g und einem mittleren Porenradius von 50 bis 2000 Å.

Nach US-PS 3 939 199 soll das Gesamtporenvolumen eines sehr leistungsfähigen Trägers bei 0,4 - 1,2 ml/g liegen, und weniger als 10 % dieses Volumens sollen von "Mikroporen" mit einem Porendurchmesser unter 30 Å (Angström) gebildet werden. Solche Träger können aus aerogenem $SiO_2$ oder einem aerogenen $SiO_2$-$Al_2O_3$-Gemisch hergestellt werden, welches in Form von glasigen Mikrokugeln vorliegt, die beispielsweise durch Flammenhydrolyse von Siliciumtetrachlorid oder eines Siliciumtetrachlorid-Aluminiumtrichlorid-Gemisches in einer Knallgasflamme hergestellt werden können. Im Handel sind diese Mikrokugeln unter dem Namen ®Aerosil oder ®Cabosil erhältlich.

In der EP-A-0 403 950 wird ein Träger der eben genannten Art beschrieben, der aus $SiO_2$ oder $SiO_2$-$Al_2O_3$-Gemisch mit einer Oberfläche von 50 - 250 $m^2$/g und einem Porenvolumen von 0,4 - 1,2 ml/g besteht und dessen Teilchen eine Korngröße von 4 bis 9 mm haben, wobei 5 bis 20 % des Porenvolumens des Trägers von Poren mit Radien von 200 bis 3000 Å und 50 bis 90 % des Porenvolumens von Poren mit Radien von 70 bis 100 Å gebildet wird.

Aus den Mikrokugeln werden dann vorteilhafterweise gemäß EP-A-0 431 478 unter Zusatz von einem oder mehreren Carboxylaten von Li, Mg, Al, Zn, Fe oder Mn als Bindemitteln und unter Zusatz von organischen Füllstoffen (wie Zucker, Harnstoff, höheren Fettsäuren, längerkettigen Paraffinen, mikrokristalliner Cellulose) und Gleitmitteln (wie Kaolin, Graphit, Metallseifen), Formkörper, d.h. geformte Trägerteilchen, z.B. durch Tablettieren oder Extrudieren, hergestellt. Anschließend werden die Formkörper in $O_2$-haltigen Gasen bei etwa 500 - 900°C für etwa 0,25 - 5 Stunden geglüht.

Die katalytisch aktiven Substanzen werden in üblicher Weise auf den Träger aufgebracht, beispielsweise durch Tränken des Trägers mit einer Lösung der aktiven Substanzen, anschließende Trocknung und gegebenenfalls Reduktion. Jedoch kann man die aktiven Substanzen auch beispielsweise durch Ausfällung auf dem Träger, durch Aufsprühen, Aufdampfen, Tauchen aufbringen.

Als Lösungsmittel für die katalytisch aktiven Substanzen sind vor allem unsubstituierte Carbonsäuren mit 2 bis 10 Kohlenstoffatomen, wie Essigsäure, Propionsäure, n- und iso-Buttersäure und die verschiedenen Valeriansäuren geeignet. Wegen ihrer physikalischen Eigenschaften und auch aus wirtschaftlichen Gründen wird vorzugsweise Essigsäure als Lösungsmittel eingesetzt. Die zusätzliche Verwendung eines inerten Lösungsmittels ist dann zweckmäßig, wenn die Substanzen in der Carbonsäure nicht ausreichend löslich sind. So läßt sich z.B. Palladiumchlorid in einer wäßrigen Essigsäure wesentlich besser lösen als in Eisessig. Als zusätzliche Lösungsmittel kommen diejenigen in Betracht, die inert und mit der Carbonsäure mischbar sind, zum Beispiel Wasser und Ether, wie Tetrahydrofuran oder Dioxan, aber auch Kohlenwasserstoffe, wie Benzol.

Die Tränkung des Katalysatorträgers mit der Lösung der aktiven Komponenten wird vorzugsweise so vorgenommen, daß das Trägermaterial mit der Lösung überschichtet und die überschüssige Lösung dann abgegossen oder abfiltriert wird. Mit Rücksicht auf Lösungsverluste ist es vorteilhaft, nur die dem integralen Porenvolumen des Katalysatorträgers entsprechende Menge an Lösung einzusetzen und sorgfältig durchzumischen, damit die Teilchen des Trägermaterials gleichmäßig benetzt werden. Diese Durchmischung läßt sich z.B. durch Rühren erreichen. Es ist zweckmäßig, den Tränkungsvorgang und das Durchmischen gleichzeitig durchzuführen, beispielsweise in einer Drehtrommel oder einem Taumeltrockner, wobei sich die Trocknung sofort anschließen kann. Weiterhin ist es zweckmäßig, die Zusammensetzung der zum Tränken des Katalysatorträgers verwendeten Lösung so zu bemessen, daß durch einmaliges Tränken die gewünschte Menge aktiver Stoffe aufgebracht wird. Man kann sie jedoch auch durch mehrere Imprägnierungsschritte aufbringen.

Die Trocknung des mit dieser Lösung getränkten Katalysatorträgers wird vorzugsweise unter vermindertem Druck durchgeführt. Die Temperatur bei der Trocknung sollte unter 120°C, vorzugsweise unter 90°C liegen. Weiterhin empfiehlt es sich im allgemeinen, die Trocknung in einem Inertgasstrom, beispielsweise in einem Stickstoff- oder Kohlendioxidstrom vorzunehmen. Der Lösungsmittel-Restgehaltnach der Trocknung sollte vorzugsweise weniger als 8 Gew.-%, insbesondere weniger als 6 Gew.-% betragen.

Der Katalysator enthält als Edelmetallkomponente Palladium und/oder dessen Verbindungen.

Als Verbindungen des Palladiums kommen alle Salze und Komplexe in Betracht, die löslich (sowie gegebenenfalls reduzierbar) sind und im fertigen (wenn nötig, mit Wasser gewaschenen) Katalysator keine desaktivierenden Stoffe wie Halogen oder Schwefel hinterlassen. Besonders geeignet sind die Carboxylate, vorzugsweise die Salze der aliphatischen Monocarbonsäuren mit 2 bis 5 Kohlenstoffatomen, etwa das Acetat, das Propionat oder das Butyrat. Weiter sind beispielsweise geeignet das Nitrat, Nitrit, Oxidhydrat, Oxalat, Acetylacetonat, Acetoacetat. Wegen seiner Löslichkeit und seiner Zugänglichkeit ist Palladiumacetat die besonders bevorzugte Palladiumverbindung.

Im allgemeinen liegt der Gehalt an Palladium im Katalysator bei 0,3-3 Gew.-%, vorzugsweise bei 1,5 bis 2,5 Gew.-%, insbesondere bei 2-2,5 Gew.-%.

Als Aktivatoren enthält der Katalysator mindestens eine Bariumverbindung, vorzugsweise ein Carboxylat wie Formiat, Acetat, Propionat, Butyrat. Weiterhin ist mindestens eine Alkaliverbindung anwesend, vorzugsweise mindestens eine K-, Rb- oder Cs-Verbindung, insbesondere mindestens eine K-Verbindung. Geeignet sind vor allem Carboxylate als Alkaliverbindungen, insbesondere Acetate und Propionate.

Geeignet sind als Barium- bzw. Alkaliverbindungen auch solche, die unter Reaktionsbedingungen in das Acetat übergehen, wie etwa Hydroxide, Oxide, Carbonate.

Im allgemeinen liegt der Gehalt an Barium im Katalysator bei 0,1-10 Gew.-%, vorzugsweise bei 0,2-4 Gew.-%, insbesondere bei 0,4-3 Gew.-%. Der Gehalt an Alkalielementen beträgt im allgemeinen 0,3-10 Gew.-%, vorzugsweise 0,5-8 Gew.-%, insbesondere 1-4 Gew.-%. Die angegebenen Prozentzahlen betreffen stets die auf dem Katalysator anwesenden Mengen der Elemente Palladium, Barium, Alkalimetall, bezogen auf die Gesamtmasse des Katalysators; etwaige Anionen werden nicht mitgerechnet.

Falls eine Reduktion der Palladiumverbindung durchgeführt wird, was manchmal nützlich sein kann, so kann diese im Vakuum, bei Normaldruck oder bei erhöhtem Druck bis zu 10 bar, ausgeführt werden. Dabei empfiehlt es sich, das Reduktionsmittel um so stärker mit einem Inertgas zu verdünnen, je höher der Druck ist. Die Reduktionstemperatur liegt i. allg. zwischen 40 und 260°C, vorzugsweise zwischen 70 und 200°C. Im allgemeinen ist es zweckmäßig, für die Reduktion ein Inertgas-Reduktionsmittel-Gemisch zu verwenden, das 0,01 bis 50 Vol.-%, vorzugsweise 0,5 bis 20 Vol.-% Reduktionsmittel enthält. Als Inertgas können beispielsweise Stickstoff, Kohlendioxid oder ein Edelgas verwendet werden. Als Reduktionsmittel kommen beispielsweise Wasserstoff, Methanol, Formaldehyd, Ethylen, Propylen, Isobutylen, Butylen und andere Olefine in Frage. Die Menge des Reduktionsmittels richtet sich nach der Menge des Palladiums; das Reduktionsäquivalent sollte i. allg. mindestens das 1- bis 1,5-fache des Oxydationsäquivalents betragen, jedoch schaden größere Mengen Reduktionsmittel nicht. Die Reduktion kann im Anschluß an die Trocknung in der gleichen Anlage vorgenommen werden.

Die Herstellung des Vinylacetats erfolgt im allgemeinen durch Leiten von Essigsäure, Ethylen und Sauerstoff oder Sauerstoff enthaltenden Gasen bei Temperaturen von 100 bis 220°C, vorzugsweise 120 bis 200°C, und bei Drücken von 1 bis 25 bar, vorzugsweise 1 bis 20 bar, über den fertigen Katalysator, wobei nicht-umgesetzte Komponenten im Kreis geführt werden können. Zweckmäßig hält man die Sauerstoffkonzentration unter 10 Vol.-% (bezogen auf das essigsäurefreie Gasgemisch). Unter Umständen ist jedoch auch eine Verdünnung mit inerten Gasen wie Stickstoff oder Kohlendioxid vorteilhaft. Besonders $CO_2$ eignet sich zur Verdünnung bei Kreisprozessen, da es in geringen Mengen während der Reaktion gebildet wird.

Die folgenden Beispiele sollen die Erfindung erläutern. Die Prozentangaben der Elemente Pd, Ba und K sind Gewichtsprozente.

Vergleichsbeispiel 1

100 g Kieselsäure-Tabletten, die aus aerogenen $SiO_2$-Mikrokugeln mit einer Oberfläche von 200 $m^2/g$ durch Verpressen mit Mg-Stearat als Bindemittel und nachfolgendem Ausglühen gemäß EP-A-0 431 478 hergestellt worden waren, wurden mit einer Lösung von 5,6 g Pd-Acetat, 5,1 g Cd-Acetat und 5,5 g K-Acetat in 78,5 ml Eisessig getränkt und getrocknet. Der fertige Katalysator enthielt 2,3 % Pd, 1,8 % Cd und 1,9 % K, bezogen auf das aus Salzen und Träger bestehende System.

Es wurden 50 ml des fertigen Katalysators in ein Reaktionsrohr von 8 mm Innendurchmesser und einer Länge von 1,5 m eingefüllt. Dann wurde bei einem Druck von 8 bar (Reaktoreingang) und einer Katalysatortemperatur von 150°C das umzusetzende Gas über den Katalysator geleitet. Dieses Gas bestand am Reaktoreingang aus 27 Vol-% Ethylen, 55 Vol.-% $N_2$, 12 Vol.-% Essigsäure und 6 Vol.-% $O_2$. Die Ergebnisse sind aus der Tabelle ersichtlich.

Vergleichsbeispiel 2

Es wurde gearbeitet wie im Vergleichsbeispiel 1, mit der Ausnahme, daß Al-Stearat anstelle von Mg-Stearat als Bindemittel benutzt wurde. Die Ausprüfung unter den Bedingungen von Vergleichsbeispiel 1 ergab die in der Tabelle aufgeführten Werte.

Beispiel 1

Es wurden 100 g des in Vergleichsbeispiel 1 genannten Trägers mit einer Lösung von 5,6 g Pd-Acetat, 5,3 g Ba-Acetat und 5,5 g K-Acetat in 78,5 ml Eisessig getränkt und getrocknet. Der fertige Katalysator enthielt 2,3 % Pd, 2,2 % Ba und 1,9 % K, bezogen auf das aus Träger und Salzen bestehende System. Unter den Bedingungen von Vergleichsbeispiel 1 ergaben sich die in der Tabelle aufgeführten Werte.

Beispiel 2

Der in Vergleichsbeispiel 1 verwendete Träger wurde mit einer Lösung von 5,6 g Pd-Acetat, 1,1 g Ba-Acetat und 5,5 g K-Acetat in 78,5 ml Eisessig getränkt und getrocknet. Der fertige Katalysator enthielt 2,3 % Pd, 0,5 % Ba und 1,9 % K, bezogen auf das aus Träger und Salzen bestehende System. Die unter den Bedingungen von Vergleichsbeispiel 1 erzielten Werte sind in der Tabelle aufgeführt.

Tabelle

| | Vergleichsbeispiel | | Beispiel | |
|---|---|---|---|---|
| | 1 | 2 | 1 | 2 |
| RZA (g/l • h) | 838 | 790 | 840 | 780 |
| Verbrennung (%) | 5,4 | 4,6 | 6 | 4 |
| Ethylacetatgehalt (ppm) | 190 | 218 | 20 | 80 |
| "RZA" bedeutet die Raum-Zeit-Ausbeute; "Verbrennung (%)" bedeutet den Prozentsatz des umgesetzten Ethylens, der in $CO_2$ umgewandelt wird; "Ethylacetatgehalt" bezieht sich auf den Gehalt des kondensierten Teils des Reaktionsproduktes an Ethylacetat. | | | | |

**Patentansprüche**

1. Verfahren zur Herstellung von Vinylacetat in der Gasphase aus Ethylen, Essigsäure und Sauerstoff oder Sauerstoff enthaltenden Gasen an einem Katalysator, der Palladium und/oder dessen Verbindungen sowie Alkaliverbindungen auf einem Träger enthält, dadurch gekennzeichnet, daß der Katalysator mindestens eine Bariumverbindung enthält und daß Gold und Cadmium und deren Verbindungen abwesend sind.

2.  Verfahren nach Anspruch 1, dadurch gekennzeichnet, daß der Katalysator mindestens eine Kaliumverbindung enthält.

3.  Verfahren nach Anspruch 1 oder 2, dadurch gekennzeichnet, daß der Katalysator 0,1 Gew.-% bis 10 Gew.-% Barium, bezogen auf die Gesamtmasse des Katalysators enthält.

4.  Verfahren nach einem der Ansprüche 1 bis 3, dadurch gekennzeichnet, daß der Katalysator 0,2 Gew.-% bis 4 Gew.-% Barium, bezogen auf die Gesamtmasse des Katalysators enthält.

5.  Katalysator, der Palladium und/oder dessen Verbindungen sowie Alkaliverbindungen auf einem Träger enthält, dadurch gekennzeichnet, daß der Katalysator mindestens eine Bariumverbindung enthält und daß Gold und Cadmium und deren Verbindungen abwesend sind.

6.  Katalysator nach Anspruch 5, dadurch gekennzeichnet, daß er mindestens eine Kaliumverbindung enthält.

7.  Katalysator nach Anspruch 5 oder 6, dadurch gekennzeichnet, daß er 0,1 Gew.-% bis 10 Gew.-% Barium, bezogen auf die Gesamtmasse des Katalysators, enthält.

8.  Katalysator nach Anspruch 5 oder 6, dadurch gekennzeichnet, daß er 0,2 Gew.-% bis 4 Gew.-% Barium, bezogen auf die Gesamtmasse des Katalysators, enthält.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | FR-A-1 598 149 (NATIONAL DISTILLERS AND CHEMICAL CORPORATION) <br> * Seite 3 - Seite 7 * <br> --- | 1-4 | C07C67/055 <br> C07C69/15 <br> B01J23/58 |
| Y <br> X | US-A-4 550 097 (CHARLES A. DRAKE) <br><br> * Spalte 2, Zeile 42 - Spalte 8, Zeile 20 * <br> --- | 1-4 <br> 5-8 | |
| Y <br> X | US-A-4 321 409 (YOSHINORI YOSHIDA ET AL.) <br><br> * Spalte 3, Zeile 59 - Spalte 10, Zeile 40 * <br> --- | 1-4 <br> 5-8 | |
| Y | DE-C-1 271 681 (NATIONAL DISTILLERS AND CHEMICAL CORPORATION) <br> * Ansprüche 1-4; Beispiele 1,6 * <br> --- | 1-4 | |
| Y | US-A-3 742 039 (ISAO ONO ET AL.) <br> * Ansprüche 1,2,4-6; Beispiele 1,10 * <br> --- | 1-4 | |
| A,D | US-A-3 939 199 (HANS FERNHOLZ ET AL.) <br> * Anspruch 1; Beispiel 2 * <br> --- | 1-5 | **RECHERCHIERTE SACHGEBIETE (Int. Cl.5 )** |
| A | FR-A-1 546 361 (SOCIETA ITALIANA RESINE S.P.A.) <br> * Seite 2 - Seite 3 * <br><br> ----- | 1-4 | C07C <br> B01J |

Der vorliegende Recherchenbericht wurde für alle Patentansprüche erstellt

| Recherchenort | Abschlußdatum der Recherche | Prüfer |
|---|---|---|
| BERLIN | 17 AUGUST 1992 | RUFET J. |